(19) **Europäisches Patentamt**
**European Patent Office**
**Office européen des brevets**

(11) **EP 2 520 278 B1**

(12) **EUROPEAN PATENT SPECIFICATION**

(45) Date of publication and mention
of the grant of the patent:
**01.08.2018 Bulletin 2018/31**

(51) Int Cl.:
*A61K 8/9789* (2017.01)    *A61Q 11/02* (2006.01)

(21) Application number: **10841365.9**

(86) International application number:
**PCT/RU2010/000793**

(22) Date of filing: **27.12.2010**

(87) International publication number:
**WO 2011/081573 (07.07.2011 Gazette 2011/27)**

(54) **MEANS FOR REMOVING TOBACCO TARS AND COMPOSITION CONTAINING SAME**

MITTEL ZUR BESEITIGUNG VON TABAKTEEREN UND ZUSAMMENSETZUNG DAMIT

MOYENS D'ÉLIMINATION DES GOUDRONS DE TABAC ET COMPOSITION CONTENANT CES MOYENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2009 RU 2009148450**

(43) Date of publication of application:
**07.11.2012 Bulletin 2012/45**

(73) Proprietor: **Obshchestvo S Ogranichennoi Otvetstvennostyu**
**«Splat-Kosmetika» (OOO «Splat-Kosmetika»)**
**Moscow 107076 (RU)**

(72) Inventors:
- **BELOUS, Elena Yurievna**
  **Moscow 121309 (RU)**
- **MALTABAR, Svetlana Alekseevna**
  **Moscowskaya obl. 143005 (RU)**
- **GALIMOVA, Anna Zufarovna**
  **Moskovskaya obl. 121099 (RU)**

(74) Representative: **Glawe, Delfs, Moll**
**Partnerschaft mbB von**
**Patent- und Rechtsanwälten**
**Postfach 13 03 91**
**20103 Hamburg (DE)**

(56) References cited:
**WO-A1-2005/063186**    **WO-A2-2004/004650**
**WO-A2-2007/109802**    **JP-A- S62 181 212**
**JP-A- 2005 194 526**    **JP-A- 2008 013 445**
**RU-C1- 2 203 032**

- **CHANG CHIEHMING J ET AL: "Separation of Catechins form Green Tea Using Carbon Dioxide Extraction", FOOD CHEMISTRY, ELSEVIER LTD, NL, vol. 68, no. 1, 1 January 2000 (2000-01-01), pages 109-113, XP008090733, ISSN: 0308-8146, DOI: 10.1016/S0308-8146(99)00176-4**
- **DATABASE GNPD [Online] MINTEL; May 2009 (2009-05), Holland & Barrett: "Pomegranate Toothpaste", XP002743854, Database accession no. 1107762**
- **DATABASE GNPD [Online] MINTEL; May 2005 (2005-05), Henkel: "Perfection 3 toothpaste (vademecum)", XP002743855, Database accession no. 345240**

**Description**

FIELD OF THE INVENTION

This invention relates to cosmetology, in particular to tobacco tar removal agents on the basis of terpenes and terpenoids, and also to oral cavity hygiene compositions based on this agent, including toothpastes and other products.

STATE OF THE ART

One of the modem-day problems in dentistry is the problem of smokers teeth whitening, because in the process of smoking, teeth may change their color in a firm and lasting fashion for the reason that tobacco smoke contains such components as nicotine, tar, ammonium and phenolic compounds.

The aforesaid compounds adversely affect the enamel of the teeth causing its discoloration: there occur yellow-and-brown, and sometimes almost black, deposits that are difficult to remove. And if the smoker does not comply with the rules of individual oral cavity hygiene, then discoloration may be twice as great. Besides, tobacco tar, when deposited on the teeth, contributes to the speedy accumulation of microbial plaque, formation of dental calculus and general deterioration of the periodontium.

Tobacco smoke constitutes a concentrated aerosol: a mixture of gases and highly concentrated, air-suspended particles that condense and form the so-called tobacco tar. The tobacco smoke phase that contains solid particles includes for the most part nicotine, water and tobacco tar.

Patent research has shown that up to this time, the following agents and materials have been used for tobacco tar removal:

1. Highly abrasive substances
2. Substances containing active oxygen (peroxy acids) or peroxides and their derivatives
3. Surfactants
4. Enzymes.

Highly abrasive substances remove tar from the dental surface by means of abrasive erosion. Such substances are disclosed in multiple patents, and they can be used both in toothpastes and in other compositions for oral cavity hygiene.

For example, application of such agents is disclosed in patent application JP2003335646. The respective dental cleansing agent for the removal of stains caused by smoking contains a phytic acid-based compound and particles with an average diameter of from 0.05 to 2 mm and ultimate load of 10g/particle.

Among the disadvantages of such agents is the possibility of tooth enamel damaging.

Substances containing active oxygen and surfactants achieve the effect of teeth whitening by a chemical process during their contact with tar deposits on the tooth surface.

The chemistry of such processes is not completely studied, but it is assumed that the freed oxygen destroys tooth chromogenes by means of oxidation of unsaturated carbon-carbon, carbon-oxygen and carbon-nitrogen bonds located in discolored molecules, converting them into a bleached or soluble state.

As far as surfactants are concerned, they also enter into a reaction with the plaque in the form of a tar deposit and produce an action similar to that produced by detergents used for laundering.

Examples of agents with active oxygen and surfactants are given in the following documents.

Patent JP2002047157 (LION CORP) discloses a composition comprising (A) a non-ionic surfactant, (B) one or more substances selected from the group comprising phenoxyethanol, phenoxypropanol and phenoxyisopropanol, and (C) ethanol. According to the patent, such complex actively removes stains from the teeth, including cigarette tar; the composition also significantly boosts the effect of chemical oral cavity cleansing.

A similar composition is disclosed in patent JP2001199855 (KOBAYASHI PHARMA).

Patent JP2006104101 (LION CORP) discloses a composition for oral cavity hygiene comprising, in certain proportions, the following components: a water-soluble pyrophosphate; one or more polyvalent alcohols selected from the group comprising polyethyleneglycol, diethyleneglycol, polypropyleneglycol and others; a lauryl sulfate salt and an amphoteric betaine surfactant.

US patent 5662888 discloses a toothpaste for smokers that dissolves and removes cigarette tar from the teeth, gums, tongue and other surfaces of the oral cavity. The toothpaste comprises at least one non-ionic surfactant in a concentration range from 0.3% to 6.5% by weight, methyl salicylate dissolving tobacco tar, in an approximate concentration range from 0.06% to 0.20% by weight, at least one ether oil in an approximate concentration range from 0.30% to 2.0% by weight, ethyl alcohol in an approximate concentration range from 12.0% to 20.0% by weight, at least one anionic surfactant in a concentration range from 0.3% to 4.5% by weight, and a gelling agent.

However, all known compositions are based on the fact that the dental tar plaque is bleached but not completely removed. Besides, all the above compositions have a significant disadvantage of causing sensibilization of the majority of users of such products, the patients developing sensitivity to the action of heat, cold etc.

Enzyme preparations included in the composition of an oral cavity hygiene agent possess a fairly broad action range. Enzymes dissolve the organic material of the dental plaque without damaging living tissue, produce a beneficial effect on the tissues of periodontium and oral mucosa, utilize toxic and irritating life products of the dental plaque microorganisms, and produce, directly or indirectly, a bactericidal and bacteriostatic action. The following enzymes are usually used: proteinases, dextranases, carbohydrase, invertase, mutanase, oxireductase, lactate dehydrogenase, and amyloglucosidase.

The use of enzymes for the removal of tobacco tar plaque is disclosed in the description of a toothpaste according to patent CN1864661 (A). The toothpaste comprises, mass. %: silicon oxide nanoparticles 30-40, natural glycerin 10-15, sorbitol 18-30, methylcellulose 0.5-1.2, hydroxyethylcellulose 0.1-0.3, sodium lauryl sarcosinate 1.5-2.5, a set of enzymes 0.01-0.1, whitening particles 1.5-5, ETDA-2Na 0.1-0.3, zinc citrate 0.5-3.5, propolis 0.3-0.8, strontium chloride 0.5-0.8, saccharine 0.2-0.3, D-panthenol 0.8-1.0, flavorant 0.8-1.2 and water - the rest.

As stated in the patent, the toothpaste ensures an acceptable removal degree of the discolored plaque and does not damage enamel.

However, the use of enzymes does not ensure satisfactory removal of tar deposits.

It should be noted that, in the field of pharmaceutical and hygienic products used for dental care and oral cavity hygiene, great popularity has been gained by all kinds of additions of biologically active substances, such as plant extracts, used for a variety of purposes: e.g. patent RU2310436 discloses the use of a nutraceutical in the form of fir needle and plantain oil solutions in the toothpaste for the prevention and treatment of caries, gingivitis and periodontitis, as well as to inhibit formation and reduce the mass of the existing tartar.

For similar purposes, an agent is used in the form of a biologically active additive comprising chlorophyll-carotene fir needle paste, or Aloe-Vera extract and aqueous-alcohol, aqueous-alcohol-glycerin and $CO_2$ extracts of raw materials of plant origin (see RU2306922). The agent is included in the toothpaste composition.

The latter toothpaste comprises the following components: silicon dioxide as abrasive base, sorbitol as moisturizer, purified sodium carboxy-methylcellulose as excipient thickener, sodium monofluorophosphate as an anti-caries additive, sodium lauryl sulfate as a cleansing and foam-generating agent; a biologically active additive, sodium saccharinate, as a taste additive, a preservation agent; as target additives, it comprises a colorant and flavorant in an aqueous medium, a biologically active additive consisting of $CO_2$ extract of plant materials, chlorophyll-carotene fir needle paste, or Aloe-Vera extract and aqueous-alcohol, and aqueous-alcohol-glycerin extracts of raw materials of plant origin, and comprising, as an aqueous medium, a herb broth aqueous solution obtained by maceration of fine-cut plant raw material in the boiling bed under vacuum in an aqueous medium with the subsequent preservation with the help of an alcohol-containing composition, with the following proportions of components, mass %:

| | |
|---|---|
| Silicon dioxide | 15.0-25.0 |
| Sorbitol | 15.0-45.0 |
| Sodium carboxy-methylcellulose | 0.5-2.0 |
| Sodium monofluorophosphate | 0.5-1.14 |
| Sodium lauryl sulfate (as per100%) | 1.0-2.0 |
| biologically active additive, including | |
| chlorophyll-carotene fir needle paste or | |
| Aloe-Vera extract | 0.5-4.0 |
| aqueous-alcohol, and aqueous-alcohol-glycerin extracts | |
| of raw materials of plant origin | 1.0-5.0 |
| $CO_2$ extract of plant material | 0.01-0.1 |
| sodium saccharinate | 0.03-0.2 |
| preservation agent | 0.11-0.35 |
| Target additives, including | |
| flavorant | 0.5-1.5 |
| colorant | 0.1-0.5 |
| herb broth | up to 100 |

The foaming composition for oral cavity hygiene is disclosed in Chinese publication CN1172647 (A). This composition comprises sodium fluoride, stannous fluoride, sodium monofluorophosphate, strontium chloride, calcium lactate, ethyl linoleate, sodium lauryl sulfate, plant extracts and distilled water.

CHANG CHIEHMING J ET AL: "Separation of Catechins from Green Tea Using Carbon Dioxide Extraction" (FOOD CHEMISTRY, ELSEVIER LTD, NL, VOL 68, NO. 1) relates to the separation of catechins from green tea by supercritical extraction by natural carbon dioxide. WO 2007/109802 A2 also relates to the production of green tea extracts by supercritical extraction by natural carbon dioxide.

WO 2004/004650 A2 discloses toothpaste compositions and mouth rinse fluids, which comprise therapeutically effective amounts of limonene for inhibiting the growth of or eradicating disease-causing pathogens. The formulations disclosed in WO 2004/004650 A2 facilitate teeth whitening.

JP S62181212 A discloses an oral cavity cleansing composition comprising one or more monoterpenes and one or more polishing agents ensuring chemical and physical removal of tobacco tar.

Use of pomegranate extract as main ingredient of teeth whitening toothpastes is known in the state of the art (DATABASE GNPD [Online] MINTEL; May 2009; Holland&Barrett: "Pomegranate Toothpaste", Database accession no. 1107762).

The closest prior art to the claimed invention is JP 2005 194526 A describing a decomposing and removing agent of the colored stain comprising active components of extracts of at least one plant, including sage. The extracts are obtained by supercritical extraction by natural carbon dioxide. Also RU 2 203 032 C1 describes an agent for exogenous prevention of diseases and hygiene of the oral cavity. The agent is in the form of dentifrice (toothpaste) and comprises carbon dioxide concentrates of sage, walnut, horsetail, echinacea, rosemary as biologically active additives, obtained by supercritical extraction.

None of the analyzed technical solutions ensure effective tobacco tar removal.

The objective of the invention is effective tobacco tar removal.

The said objective is resolved by means of a tobacco tar removal agent comprising carbon dioxide plant extracts with terpene and/or terpenoid content in the extract of min. 10 mass %, obtained by supercritical extraction from plants including green tea, walnut, sage and fennel.

The agent additionally comprises carbon dioxide pomegranate extract obtained by supercritical extraction. The scope of the invention is defined by the appended claims. The agent comprises extracts in the following proportions, mass %:

| | |
|---|---|
| Pomegranate extract | 10-60 |
| Walnut extract | 10-60 |
| Sage extract | 10-60 |
| Fennel extract | 10-60 |
| Green tea extract | the rest |

The set objective is also achieved by means of a composition for oral cavity hygiene, which comprises the earlier described tobacco tar removal agent in an effective quantity, and an acceptable carrier.

In specific embodiments of the invention, the composition constitutes a toothpaste.

The claimed composition may constitute a mouth rinse composition, including foaming mouth rinse composition.

The claimed composition may constitute a chewing gum.

The set objective is achieved by means of a toothpaste for oral cavity hygiene comprising the tobacco tar removal agent in an effective quantity, and an acceptable carrier comprising substances selected from the group comprising solvents, thickeners, surfactants, abrasive substances, emulgators, solubilizers, moisturizers and mixtures thereof.

In specific embodiments of the claimed toothpaste, the carrier comprises water as solvent, silicon dioxide as abrasive substance, sodium carboxy-methylcellulose as thickener, at least one moisturizer selected from the group comprising sorbitol, glycerin and polyethyleneglycol PEG-400 as moisturizer, at least one solubilizer selected from the group comprising hydrogenated castor oil and Polysorbate-20 as solubilizer, and sodium lauryl sarcosinate as surfactant, with the following proportions of components, mass %:

| | |
|---|---|
| Tobacco tar removal agent | 0.01 - 2.0 |
| Silicon dioxide | 5.0 - 60.0 |
| Sodium carboxy-methylcellulose | 0.1 - 5.0 |
| Moisturizer | 0.5 - 70 |
| Solubilizer | 0.1 - 10.0 |
| Sodium lauryl sarcosinate | 0.5 - 10.0 |
| Water | the rest |

The toothpaste may additionally comprise at least one substance selected from the group comprising sweeteners, flavorants and preservation agents in a quantity not exceeding 10 mass %.

The toothpaste may also additionally comprise polyvinylpyrrolidon/vinyl acetate, hydroxyapatite, menthyl lactate, isopropyl methylphenol, $CO_2$ baobab plant extract, thyme ether oil, camphor oil, lavender bioconcentrate, chlorophyll copper complex, flavorants, preservation agents, stevia extract and sucralose with the following proportions of components, mass %:

| | |
|---|---|
| Tobacco tar removal agent | 0.01-2 |
| Silicon dioxide | 5-60 |
| Sodium carboxy-methylcellulose | 0.1-5 |
| Sorbitol | 0.5-60 |
| Glycerin | 0.5-60 |
| Polyethyleneglycol PEG-400 | 0.1-10 |
| Hydrogenated castor oil | 0.1-7 |
| Polysorbate-20 | 0.1-5 |
| Polyvinylpyrrolidon/vinyl acetate | max. 3 |
| Sodium lauryl sarcosinate | 0.5-10 |
| Stevia extract | max. 3 |
| Sucralose | max. 2 |
| Flavorants | max. 3 |
| Preservation agents | max. 1 |
| Hydroxyapatite | max. 20 |
| Menthyl lactate | max. 5.0 |
| Isopropyl methylphenol | max. 0.1 |
| $CO_2$ baobab plant extract | max. 3.0 |
| Thyme ether oil | max. 1.0 |
| Camphor oil | max. 1.0 |
| Lavender bioconcentrate | max. 5.0 |
| Chlorophyll copper complex | max. 5.0 |
| Water | the rest |

In another embodiment of the invention, the toothpaste may additionally comprise polyvinylpyrrolidon/vinyl acetate, hydroxyapatite, menthyl lactate, isopropyl methylphenol, $CO_2$ baobab plant extract, bergamot ether oil, anise ether oil flavorants, preservation agents, colorants, stevia extract and sucralose with the following proportions of components, mass %:

| | |
|---|---|
| Tobacco tar removal agent | 0.01-2 |
| Silicon dioxide | 5-60 |
| Sodium carboxy-methylcellulose | 0.1-5 |
| Sorbitol | 0.5-60 |
| Glycerin | 0.5-60 |
| Polyethyleneglycol PEG-400 | 0.1-10 |
| Hydrogenated castor oil | 0.1-7 |
| Polysorbate-20 | 0.1-5 |
| Polyvinylpyrrolidon/vinyl acetate | max. 3 |
| Sodium lauryl sarcosinate | 0.5-10 |
| Stevia extract | max. 3 |
| Sucralose | max. 2 |
| Flavorants | max. 3 |
| Preservation agents | max. 1 |
| Hydroxyapatite | max. 20 |
| Menthyl lactate | max. 5.0 |
| Isopropyl methylphenol | max. 0.1 |

(continued)

| | |
|---|---|
| CO$_2$ baobab plant extract | max. 3.0 |
| Bergamot ether oil | max. 1.0 |
| Anise ether oil | max. 1.0 |
| Colorant | max. 10.0 |
| Water | the rest |

The objective is also resolved by means of a foaming mouth rinse composition for oral cavity hygiene comprising the above-mentioned tobacco tar removal agent in an effective quantity, and an acceptable carrier comprising substances selected from the group comprising solvents, surfactants, foam stabilizers, emulgators, thickeners, solubilizers, moisturizers, acidity regulators, sweeteners and mixtures thereof.

In specific embodiments of the invention, the composition comprises water as solvent, sodium lauryl sarcosinate as surfactant, carrageenan as foam stabilizer, at least one solubilizer selected from the group comprising hydrogenated castor oil and Polysorbate-20 as solubilizer, sorbitol as moisturizer, polyvinylpyrrolidon/vinyl acetate as thickener, and at least one component selected from the group comprising L-arginine and citric acid as acidity regulator, with the following proportions of components, mass %:

| | |
|---|---|
| Tobacco tar removal agent | 0.01-2 |
| Sodium lauryl sarcosinate | 0.1-10 |
| Carrageenan | 0.01-3 |
| Solubilizer, | |
| selected from the group comprising: | |
| hydrogenated castor oil | 0.1-7.0 |
| Polysorbate-20 | 0.1-5.0 |
| Sorbitol | 1.0-20.0 |
| Polyvinylpyrrolidon/vinyl acetate | 0.1-3.0 |
| At least one регулятор, | |
| selected from the group comprising | |
| L-arginine | 0.01-1.0 |
| and citric acid | 0.01-5.0 |
| Water | the rest. |

The essence of the invention consists in the following.

It is known that terpenes and terpenoids are effective solvents of natural resins, the majority of which are derivatives of polycyclic hydrocarbons. For example, turpentine (terebenthene), constituting a liquid mixture of terpenes and terpenoids, dissolves certain resins, fats and oils, and is widely used for dissolving paints and varnishes.

Tobacco tar also consists mostly of polycyclic hydrocarbons. Therefore, an assumption was made that terpenes and terpenoids must dissolve tobacco tar.

When verified, the assumption held true.

For the purpose of functioning as an agent for dissolving tobacco tar with concentrated terpene content, carbon dioxide plant extracts were used comprising large quantities of terpenes and terpenoids initially.

Extracts of such plants were obtained by means of supercritical fluid extraction by natural carbon dioxide.

Extracts suitable for use according to the present invention may be obtained from any plant's portion, including leaves, stem, bark, fruit pulp, seeds, fruit skin, juice, root and mixtures thereof.

Supercritical carbon dioxide fluid extracts render it possible to achieve a higher concentration of active substances from plants compared to extracts obtained by classic methods.

To make extracts effectively usable for cleansing the teeth and oral cavity of tobacco tar, the minimal concentration of terpenes and terpenoids should be 10%. A maximum concentration may be limited only by the maximum terpene and terpenoid content in the plant in question.

For the purposes of this research, a group of plants was selected with a comparatively high terpene content, and carbon dioxide extracts were obtained from this group of plants by supercritical extraction (see table 1).

Extracts from all of the plants demonstrated high activity in respect of tobacco tar dissolution.

The best properties, availability and suitability for use in oral cavity hygiene compositions among those extracts were demonstrated by those selected from the group comprising sage extract, walnut extract, fennel extract and green tea extract.

The agent for tobacco tar dissolution may include both one extract selected from the aforesaid group, and a mixture of two or more extracts in arbitrary quantities.

It was also noted that the addition to those extracts of carbon dioxide pomegranate extract increases the tobacco tar removal rate. The pomegranate extract also comprises terpenes (terpenes upward of 3%, benzoic acid up to 0.1%, sterols 1.3%; tocopherols - 0.19%). Besides, it possesses good antioxidant action, which may also be a reason that accelerates tobacco tar dissolution.

Such choice of plants ensures the presence in the agent composition of a variety of terpenes and terpenoids, which non-linearly increase the impact produced by the extracts on tobacco tar, resulting in tar dissolution in a lesser time or fuller cleansing of the teeth.

If the agent comprises all the five extracts, then the preferred composition is a composition where each component is present in an amount of from 10 to 60 mass %, because it is in this range that the best result can be obtained.

The tobacco tar dissolution agent is one of the components of compositions for oral cavity hygiene.

Oral cavity hygiene compositions care compositions, or oral care compositions, mean, in their broadest sense, a composition comprising the above-mentioned tobacco tar dissolution agent as an active ingredient in an effective quantity, combined with an acceptable carrier.

The term "... tobacco tar removal agent ... in an effective quantity" means non-toxic but sufficient quantity of the aforesaid substances for the achievement of the desired effect.

An effective quantity may be different depending on the type of composition, particular extract or a combination thereof etc.

Such effective quantity in any individual case may be determined by any person skilled in the art by means of ordinary experimenting.

For certain embodiments of the invention (toothpaste, mousse), such quantity has been determined by the authors of the claimed invention and is described in the claims of the invention.

The acceptable carrier means a carrier rendering it possible to obtain the desired composition for oral cavity hygiene in the form of, e.g., toothpaste, mouth rinse, tooth powder, gel, mousse, chewing gum etc. In that sense, a composition according to the present invention may constitute a liquid solution of ingredients, e.g. mouth rinse, or it may be semi-hard - in the form of toothpaste or dental gel, or hard, e.g., chewing gum.

If a composition constitutes a liquid, e.g., mouth rinse, then an acceptable carrier, as a rule, constitutes an aqueous-alcohol mixture with a non-toxic alcohol (ethanol, isopropanol) content of from 5 to 30%. To reduce alcohol content in mouthwash compositions, or even completely eliminate the same, certain solubilizers may be added to the mouth rinse composition in an amount up to 10 mass%. A composition hereunder may also contain a variety of additives, e.g., moisturizers or additives improving organoleptical properties of the composition (flavorants or sweeteners).

If a composition constitutes a chewing gum, then an acceptable carrier includes synthetic or natural polymers with plastifiers, flavorants, aromatizers, preservation agents, sweeteners and other food additives.

If a composition constitutes a toothpaste, then an acceptable carrier includes solvents, thickeners, surfactants, abrasive substances, emulgators, solubilizers, moisturizers, sweeteners, preservation agents and mixtures thereof.

An analysis of the state of the art shows that for toothpastes, the abrasive substance content, as a rule, fluctuates from 5 to 60 mass %, which corresponds to the abrasive substance content in the claimed toothpaste. Abrasive substances preferred for the use in this invention include silicon dioxide-based materials, which are represented in the examples of the invention realization by silicon dioxides under Sorbosil trademark (producer: PQ Corporation) and Tixosil trademark (producer: Rhodia).

An acceptable carrier applicable for the obtaining of a composition in the form of a paste or rinsing mousse may comprise a moisturizer. A moisturizer preferably constitutes sorbitol, glycerin and/or polyethyleneglycol PEG-400; however, other moisturizers with a molecular mass in the range of 200-1000 and mixtures thereof may also be used. In known technical solutions, the concentration of the moisturizer is usually from about 0.5 to about 70 mass % of the composition. As a rule, thickeners are represented in compositions for oral cavity hygiene in an amount of up to 10 mass %. Thickeners include natural and synthetic resins and colloids. In the present invention, sodium carboxy-methylcellulose is used as thickener; besides, the functions of a thickener are performed also by the above-discussed silicon dioxide.

Any of the aforesaid compositions may additionally include any suitable flavorants or sweeteners.

Various other substances, including preservation agents, such as sodium methyl paraben and chlorophyll compounds, may be added into compositions for oral cavity hygiene according to the present invention, such as toothpastes and rinse mousses. Such auxiliary substances, in case they are present, should be introduced to the compositions in quantities that do not adversely affect the desired properties and characteristics.

Emulgators and solubilizers may be occasionally added into compositions for oral cavity hygiene. Emulgators ensure the disperse state of fats and oils in water emulsions, and solubilizers keep ingredients in the dissolved state. Solubilizers are substances that help transfer particles of other components into colloid solution.

Emulgators and solubilizers are various substances of both natural and artificial origin. In our invention, emulgators and solubilizers may, in specific embodiments of the invention, constitute balanced mixtures of mutually complementary substances, which are more effective than individual substances and are used in a variety of emulsions more often than individual substances.

Requirements to emulgators and solubilizers are similar: ensuring the stability of a composition, inertness to other components of the composition, absence of irritating action, non-toxicity, absence of disagreeable odor.

A composition in the form of toothpaste or in the form of a rinse mousse, as noted above, may comprise surfactants and, in particular, anionic surfactants, such as lauryl sulfate or sodium lauryl sarcosinate, which have multifunctional actions: solubilizing, dispersing and moisturizing. Besides, their function it is to form emulsions together with other components of compositions for oral cavity hygiene, including flavoring agents.

The effect of the surfactant presence consists in the fact that it contributes to foaming of the oral cavity hygiene composition. Compositions for oral cavity hygiene with a high degree of foaming are preferred by consumers for the reason that, according to literature, foaming contributes to the creation of a sense of effective oral cavity cleansing.

Beside the aforesaid substances, compositions may comprise a variety of auxiliary additives of plant origin in the form of extracts - propylenglycol, aqueous-alcohol, glycerin, carbon dioxide and other concentrates; ether oils and others. Such components are not basic, however they are active components. Their presence in the composition of a paste or a mousse is not mandatory, however, they impart a number of additional useful properties to the composition if present, e.g., anti-bacterial, anti-inflammatory etc.

Compositions for oral cavity hygiene according to the present invention may be obtained by mixing the ingredients. For example, a mouth rinse is obtained by dispersing an agent consisting of carbon dioxide extracts with high terpene content in a mixture of solubilizers and water, with target additives (flavorants, preservation agents, sweeteners etc.) being added thereafter.

Table 2 shows substances used in the production of pastes and mousses, their functions in the claimed compositions, and proportions of ingredients in such pastes and mousses.

Example 1.

The tobacco tar removal agent hereunder was produced by mixing five plant extracts in various proportions and combinations, with the terpene and terpenoid content in the extracts being upwards of 10%.

The extracts were obtained by means of supercritical fluid extraction by natural carbon dioxide, in the absence of inorganic salts, without solvent residues, heavy metals or reproducible microorganisms.

Table 3 shows various compositions of the tobacco tar removal agent.

To obtain comlpex compositions, comprising at least two components, mechanical mixing of extracts was conducted, e.g., with the help of an anchor mixer. Research has shown that terpene and terpenoid content in such extracts amounted to 10-80 mass %.

Example 2.

To produce toothpaste, the agent having composition C, comprising five extracts in equal quantities, was used as a tobacco tar removal agent. The toothpaste whose compositions are shown in table 2, was produced as follows: moisturizers, e.g. glycerin, sorbitol, or polyethyleneglycol, were dispersed in water under stirring in a conventional mixer. To the dispersion were added thickeners, a sweetener, a preservation agent, any salts belonging to active components, and foam stabilizers. To the gel phase were added colorants and a pigment, e.g. $TiO_2$. If the paste was not intended to be in the form of a gel, then any acid or base was added necessary to regulate pH. Such ingredients were mixed to obtain a homogeneous phase. Then the mixture was transferred to a high-speed vacuum mixer, where a silicon dioxide-based inorganic thickener was added to the mixture, then, consecutively, a silicon dioxide-based abrasive substance with other abrasive substances to be used in the composition, plant extracts and ether oils, flavorants and surfactants. Any water-insoluble antibacterial agent was solubilized in PEG-400 and introduced together with the moisturizer. Extracts containing terpenes and terpenoids were mixed with a solubilizer or mixture thereof (Polysorbate-20, PEG-40 hydrogenated castor oil) in advance. The product obtained in all cases was a homogeneous semi-hard paste or gel product.

Example 3.

To produce a foam rinse composition for oral cavity hygiene, the agent having composition C, comprising five extracts in equal quantities, was used as a tobacco tar removal agent.

The foaming composition for rinsing oral cavity according to the present invention was produced as follows.

Aqueous solutions of a moisturizer with a flavorant, organic thickener with licorice extract, and a preservation agenta, were prepared. The obtained solutions and the remaining components were consecutively mixed under 40-45°C; the last component to introduce being the surfactant. Then the obtained solution was cooled to 30-35°C, and Sebomin SB12 and Sederma were added (lactoferrin and lactoperoxidase, glucose oxidase, glucose pentaacetate and potassium thiocyanate).

The obtained pastes and mousses were tested as follows.

The objective of the test was to assess the cleansing effect of the compositions under study, determine their anti-inflammatory action and evaluate the whitening effect.

The toothpaste compositions shown in table 2 were named PRO-WHITER CLASSIC and PRO-WHITER LITE, and the mousse was labeled PRO-WHITER.

Study materials and methods. 100 smoking patients aged between 18 and 55 took part in the study. After an initial stomatological examination, the patients were divided into four groups equal in terms of quantity, age and sex composition and average stomatological status indicators.

Group #1 (prevention) - 25 patients using Pro-Whiter Lite toothpaste;
Group #2 (prevention) - 25 patients using Pro-Whiter Classic toothpaste;
Group #3 (prevention) - 25 patients using Pro-Whiter Classic toothpaste and dental mousse;
Group #4 (control) - 25 patients using a placebo toothpaste.

Prior to the start of the study, all patients were trained in the tooth brushing technique. Prevention group participants were supplied with identical toothbrushes of average hardness (Oral-B classic) and a Pro-Whiter oral cavity hygiene aid. Patients were recommended to brush their teeth twice a day (in the morning and at night) for at least 3 minutes each time, using Pro-Whiter Lite toothpastes (group #1) and Pro-Whiter Classic toothpastes (groups #2 and #3). Additionally, following each tooth brushing, group 3 participants were to apply the cleansing mousse to the surface of their teeth and gums.

Control group patients used identical toothbrushes of average hardness (Oral-B classic) and a placebo toothpaste for tooth brushing.

In the process of control stomatological examinations, the state of oral cavity hygiene, dental hard tissues and oral mucosa was assessed according to the following criteria.

Oral cavity hygienic state determination

Patient Hygiene Performance (PHP) index determination (Podshadley, Haley, 1968)

With the help of a solution for dental plaque indication, the presence thereof was identified and localization on vestibular and lingual surfaces determined.

In case of the unavailability of the index tooth, neighboring ones were examined within the namesake tooth group. Artificial crowns and parts of fixed prostheses were accounted for in the same manner as teeth. The surface of each tooth was notionally divided into 5 sectors: 1 - medial, 2 - distal, 3 - mid-occlusal, 4 - central, 5 - mid-cervical.

Dental plaque assessment codes and criteria: 0 - absence of discoloration, 1 - discoloration identified.

PHP index calculation: codes for each tooth were determined by summing up codes for each sector. To calculate the index, the following formula was used: PHP = (sum total of PHP points) / n, where n is the number of teeth (usually 6).

Interpretation of results:

| Index | Oral cavity hygiene level |
|---|---|
| 0 | excellent |
| 0.1-0.6 | good |
| 0.7-1.6 | satisfactory |
| above 1.7 | unsatisfactory |

Approximal Plaque Index (API) determination (Lange D.E., Plagmann H., 1977).

With the help of a solution for dental plaque indication, the presence thereof in interdental spaces was identified:

- I-st (upper right hand) and III-d (lower left hand) quadrants - from the oral surface;

- II-nd (upper left hand) and IV-th (lower right hand) quadrants - from the vestibular surface.

Assessment criteria:

0 points - no plaque in the interdental space (no discoloration);

9

1 point - plaque in the interdental space identified.

Index calculation: API = (total points : number of teeth) x 100%

Interpretation of results:

| Index | Oral cavity hygiene level |
|---|---|
| 25% | optimal |
| 25-39% | satisfactory |
| 40-69% | unsatisfactory |
| 70-100% | unacceptable |

At the start of the study, the oral hygienic condition of all study participants was evaluated as unsatisfactory. Differences of average PHP and API values among the groups at the initial examination were non-valid ($p > 0.5$).

Following 6 weeks of regular Pro-Whiter use, a reliable decrease of PHP values was observed in groups #1 and #2 ($p < 0.05$) and in group #3 ($p < 0.01$). The cleansing effect was from 39.2% to 45.9% and was the greatest in group #3, where Pro-Whiter Classic toothpaste and dental mousse were used in combination (table 4 - PHP dynamics).

In the period of study, the prevention groups also demonstrated a reliable decrease of the amount of dental plaque on the approximal tooth surfaces compared to initial ($p < 0.001$ - in groups #1 and #2, and $p < 0.01$ - in group #3). API values declined by 42.2% to 47.4%, with no significant differences in the cleansing effect between groups #2 and #3 (table 5 - API dynamics). In the control group, no reliable changes in hygiene indices occurred during the study ($p > 0.5$). The amount of dental plaque declined by 9.2% on smooth dental surfaces and by 10.6% on approximal surfaces. Assumably, certain improvement of the oral hygiene condition in that group may be attributed to the increased patient motivation for oral care.

In 51 (68%) patients of the prevention groups, a reduction was recorded of the hard pigmented deposit ("smoker's plaque") after the use of the Pro-Whiter oral care products; in 9 (12%) participants such smoker's plaque was not identifiable by the end of the study.

The best whitening effect was recorded in group #3, who used Pro-Whiter Classic and dental cleansing mousse. In 7 (28%) patients of this group, tooth whitening by 1-1.5 shades was observed by the Vita scale. In group #2 who used Pro-Whiter Classic toothpaste, tooth color changes by the Vita scale were recorded in 3 (12%) patients, in group #1 (Pro-Whiter Lite toothpaste) - in 2 (8%) patients.

The patients of the control group did not demonstrate any changes in the amount of the smoker's plaque and color of the teeth by the end of the study.

In addition to the above, an assessment of periodontium tissue condition was conducted by determining the Gingival Index (GI), the Sulcus Bleeding Index (SBI) and sensitivity of dental hard tissues.

The GI index was determined as follows: gingival (gum) condition was examined visually and with the help of a periodontal probe in the vicinity of 6 teeth in 4 sectors: distal, medial, at the center of the vestibular and lingual departments.

Assessment criteria: **0** - no inflammation
**1** - slight inflammation in the gum (slight discoloration, no bleeding during probing)
**2** - moderate inflammation of the gum (moderate hyperemia, edema, bleeding during probing)
3- expressed inflammation of the gum (expressed hyperemia, edema, tendency to spontaneous bleeding)

Index calculation:

$$\text{Tooth GI} = \text{total points} /4$$

$$\text{Person GI} = \text{total of teeth GI's} /n, \text{ where n is the number of the teeth (usually 6)}$$

Index interpretation:

| Index value | Criteria |
|---|---|
| 0.1-1.0 | slight gingivitis |
| 1.1-2.0 | moderate gingivitis |
| 2.1-3.0 | severe gingivitis |

The SBI index was determined as follows: probing of the sulcus of each tooth was conducted with the help of a periodontal probe:

- I-st (upper right hand) and III-d (lower left hand) quadrants - from the oral surface;
- II-nd (upper left hand) and IV-th (lower right hand) quadrants - from the vestibular surface.

Assessment criteria:

0 points - no bleeding;
1 point - sulcus bleeding.

Index calculation:

$$SBI = \frac{Total\ points}{number\ of\ teeth} \times 100\%$$

Interpretation of results:

| Index | Oral cavity hygiene level |
|---|---|
| 10% | acceptable |
| above 10% | unacceptable |

At the start of the study, signs of periodontium tissue inflammation was recorded in the majority of patients in the form of gum bleeding, gum discoloration and structural deterioration (edema) of various severity.

Average GI values did not reliably differ among the groups at the start of the study (p>0.5).

Following 6 weeks of regular Pro-Whiter Classic toothpaste use, a reliable (p<0.01) decrease of GI index in group #2 was observed. Comparable results were obtained in group #3, where Pro-Whiter Classic toothpaste and dental mousse were used in combination. Anti-inflammatory performance in these groups was 53.2% to 55.9% (table 6).

Besides, in these groups a reliable decline of sulcus bleeding by the SBI index was recorded, with the largest decrease recorded in group #3, where the gum bleeding indicator declined by 54.3% (table 7).

In group #1 (Pro-Whiter Lite toothpaste), GI and SBI values also decreased compared to initial (by 43.5% and 34.4%, respectively), however the changes were statistically non-valid (p>0.5).

The obtained results testify to the high performance of the proposed agent concerning tobacco tar removal in compositions for oral cavity hygiene, such as toothpastes and cleansing dental mousses, and render it possible to make the following conclusions.

The whitening effect of the aforesaid compositions for oral cavity hygiene is achieved by means of effective removal of the hard pigmented deposit. The majority of prevention group participants demonstrated a significant reduction of the amount of the smoker's plaque compared to initial. Besides, teeth whitening by 1-1.5 shades by the Vita scale was observed in a number of cases.

The claimed oral care compositions possess mild whitening and polishing action without damaging enamel surface.

The use of the aforesaid compositions for the purpose of oral cavity hygiene contributes to a statistically valid improvement of oral hygienic condition. Over the 6 weeks of their use, dental plaque reduction by 39.2%-45.9% was identified on smooth dental surfaces (by PHP index), and by 42.2% - 47.4% on approximal surfaces (by API index).

Over the period of study, an expressed reduction was observed in the degree of gum inflammation measured by the GI index: by 43.5% to 55.9% compared to the initial data. Besides, when used regularly, the claimed toothpastes and mousses contributed to a significant reduction of sulcus bleeding measured by the SBI index: by 34.4% to 54.3%.

The use of claimed compositions for the purposes of oral cavity hygiene contributed to the reduction of tooth sensitivity in response of thermal or tactile stimuli.

The toothpastes and cleansing dental mousse possess good organoleptic properties and persistent deodorant action. No incidents of locally irritative and allergenic action of such products on oral mucosa were identified in the period of the study.

Thus, Pro-Whiter Lite and Pro-Whiter Classic toothpastes and cleansing dental mousse may be recommended for daily oral care to adults with a habit of smoking.

Table 1.

| er. # | Extract | Composition (terpenoids, terpenes), mass %: |
|---|---|---|
| | Sage (herb) | Terpenoids - up to 60% (camphor-6.9; borneol-1.4; isothujon-6.9%; cariofillen-3%; ledol-6%; epimanool-40.9%); steroids up to 7%; tocopherols - 2.15% |
| | Rosemary (herb) | Terpenes and terpenoids up to 30.3% (borneol, caren, camphor, verbenol, cariofillen, cadinene, steroids), wax up to 24% |
| | Carrot (fruit) | Terpenes up to 16%, flavonoids - up to 9%, sterols up to 2% |
| | Hawthorn (fruit) | Triterpenes up to 14%, tocopherols - up to 1%, carotenoids - up to 80 mg% |
| | Walnut (leafe) | Terpenoids up to 38% (quinones - up to 20%), sterols - 4.4%, tocopherols - 0.6% |
| | Parsley (fruit) | Terpenes -up to 23%, flavonoids (elemicin, apiole, germacrone)-up to 50%, tocopherols - up to 1% |
| | Green tea (leafe) | Terpenoids - up to 25% (katechines) |
| | Yarrow (herb) | Terpenoids - up to 24% (incl. flavonoids), sterols -12.3%; wax - 38.6% |
| | Absinthe (herb) | Terpenes and terpenoids - up to 19% (flavonoids - up to 9%); steroids - 11.2%; A and E vitamins- up to 1%; wax up to 60% |
| | Fennel (fruit) | Terpenes - up to 50%; terpenoids - up to 10%, tocopherols - up to 0.4% |

Table 2

| Ingredient | Toothpaste | | | Dental mousse | | Function | Component significance |
|---|---|---|---|---|---|---|---|
| | Content range, mass % | PRO-WHITE R | PRO-WHITER LITE | Content range, mass % | PRO-WHITER | | |
| Tobacco tar removal agent | 0.01-2% | 0.25 | 0.25 | 0.01-2% | 0.1 | Tobacco tar dissolution | Active component, mandatory |
| Hydroxyapatite 15% | max. 20 | 3.3 | 3.3 | | | Enamel-hardening agent, reduces dental sensitivity | Active component, optional |
| Sorbitol | 0.5-60 | 15 | 15 | 1-20% | 2.0 | Moisturizer Affects composition texture, imparting it special softness and plasticity. | Basic component |
| Glycerin | 0.5-60 | 10 | 10 | | | Moisturizer Contributes to obtaining plastic thixotropic mass, stabilizes foam, improves toothpaste organoleptics | Basic component |

(continued)

| Ingredient | Toothpaste | | | Dental mousse | | Function | Component significance |
|---|---|---|---|---|---|---|---|
| | Content range, mass % | PRO-WHITE R | PRO-WHITER LITE | Content range, mass % | PRO-WHITE R | | |
| Polyethylene glycol PEG-400 | 0.1-10 | 3.5 | 3.5 | | | Moisturizer Regulates viscosity, functions as emulsion stabilizer | Basic component |
| Sodium carboxy-methylcellulose | 0.1-5,0 | 1.1 | 1.1 | | | Thickener, structurant | Basic component, but function optional |
| Sodium methyl paraben | max. 0.8 | 0.25 | 0.25 | max. 0,8 | | Preservation agent | Basic component, but function optional |
| Stevia extract | max. 3 | 0.2 | 0.2 | max. 3 | 0.15 | Sweetener | Basic component |
| Sucralose | max. 2 | 0.08 4 | 0.084 | max. 2 | 0.01 | Sweetener | Basic component |
| PLASDONES 630 (polyvinylpyr rolidon /vinyl acetate) | max. 3 | 0.3 | 0.3 | 0.1-3% | 1.5 | Stabilizer of emulsions and systems in general, e.g., with a large number of active components. Thickener and gel forming agent. | Basic component |
| Silicon dioxide | 1-60% | 29.5 | 28 | | | Abrasive substance and thickener | Basic component |
| Flavorant | max. 3 | 1 | 1 | max. 3% | 0.4 | Imparts odor and taste to compositions, supplants odor and taste of basic components. | Basic component, but function optional |
| Menthyl lactate | max. 5 | 1.5 | 1 | max. 5% | 2.0 | Possesses strong cooling effect, ensures lasting feeling of freshness, gives off no odor. Produces slight anti-inflammatory action | Basic but function optional |
| Menthol crystals | | | | max. 1% | 0.07 | Cooling and refreshing agent | Basic, but function optional |
| PEG-40 hydrogenated castor oil | 0.1-10% | 0.5 | 0.5 | 0.1-7% | 1.0 | Solubilizer (solvent) of fragrant substances and weakly polar cosmetic oils and fats | Basic component |
| Polysorbate-20 | 0.1-10% | 0.5 | 0.5 | 0.1-5% | 0.1 | Emulgator and solubilizer (solvent) of fats, ether and fragrant oils | Basic component |

(continued)

| Ingredient | Toothpaste | | | Dental mousse | | Function | Component significance |
|---|---|---|---|---|---|---|---|
| | Content range, mass % | PRO-WHITE R | PRO-WHITER LITE | Content range, mass % | PRO-WHITE R | | |
| $CO_2$ baobab plant extract | max. 3 | 0.05 | 0.05 | | | Antioxidant, moisturizing, regenerative, anti-inflammatory action. | Active component, optional |
| Propylenglycol buckthorn extract | | | | max. 10% | 1.0 | Ceratoplastic, epithelizing, analgesic, anti-inflammatory action | Active component, optional |
| Propylenglycol pomegranate extract | | | | max. 10% | 2.0 | Anti-inflammatory, stimulating, generally tonic, wound healing action | Active component, optional |
| Biosol (isopropyl methylphenol ) | max. 0.1 | 0.1 | 0.1 | | | Anti-bacterial effect | Active component, optional |
| Sodium lauryl sarcosinate 30% | 0.5-10 | 6 | 6 | 0.1-10% | 2.0 | Surfactant | Basic component |
| Carrageenan | | | | 0.01-3% | 0.2 | Foam stabilizer | Basic component |
| Thyme ether oil | max. 1 | 0.01 | | | | Aseptic, bactericidal, scar-forming, tonic, antifungal action | Active component, optional |
| Neroli ether oil | | | | max. 1% | 0.02 | Healing and aseptic agent, improves blood microcirculation | Active component, optional |
| Camphor oil | max. 1 | 0.0015 | | | | Aseptic, analgesic, stimulating, anti-inflammatory agent; improves blood circulation. Effective for treating bronchitis and colds. | Active component, optional |
| Lavender Bioconcentrate | max. 5 | 0.2 | | | | Strong aseptic action, effective for inhalations for treating colds, sinusitis and other breathing problems. | Active component, optional |
| Aloe Vera gel 10:1 | | | | max. 5 | 1.0 | Moisturizing, anti-inflammatory, sedative, softening and regenerating agent | Active component, optional |

(continued)

| Ingredient | Toothpaste | | | Dental mousse | | Function | Component significance |
|---|---|---|---|---|---|---|---|
| | Content range, mass % | PRO-WHITE R | PRO-WHITER LITE | Content range, mass % | PRO-WHITE R | | |
| Copper Chlorophyll Extract | max. 0.8 | 0.035 | | | | Chlorophyll copper complex is used instead of pure chlorophyll, as the latter is unstable. Healing, deodorating, regenerating properties. Colorant. | Active component, optional |
| Candurin Red Amber. Merc | max. 10.0 | | 0.02 | | | Mica and ferrous oxide - colorant, imparts slight radiance to toothpaste | Basic component, but function optional |
| Bergamot ether oil | max. 1.0 | | 0.05 | | | Analgesic, anti-depression, aseptic, deodorating, wound healing, stimulating, antifungal action | Active component, optional |
| Anise ether oil | max. 1.0 | | 0.05 | | | General tonic used in colds | Active component, optional |
| L-arginine (Ajinomoto) | | | | 0.01-1% | 0.25 | Acidity regulator | Basic, but function optional |
| Citric acid, dry | | | | 0.01-5% | 0.04 | Acidity regulator | Basic component |
| Sebomin SB12, Sederma | | | | max. 3.0 | 0.3 | Lactoferrin, lactoperoxidase, glucose oxidase, glucose pentaacetate and potassium thiocyanate | Active component, optional |
| LICORICE EXTRACT A.M. (licorice extract) | | | | max. 10 | 0.07 | Anti-allergic, anti-inflammatory, wound healing, anti-caries, anti-bacterial action | Active component, optional |
| Trilon BD (Na2 EDTA) | | | | max. 10 | 0.1 | Stabilizer: bonds Ca and Mg to form a residue | Basic component, optional |
| Purified water | the rest | | | | | Solvent | Basic component |

Table 3

| Input raw materials | Extract type | Tobacco tar agent compositions, mass %: | | | | |
|---|---|---|---|---|---|---|
| | | A | B | C | D | E |
| Fennel (Foeniculu m vulgare Mill.- herb) | Oily mass of brownish swamp color with a strong characteristic odor. | 100 | - | 20 | 20 | 40 |
| Green tea (Camellia sinensis - leafe) | Thick oily mass of grayish-green color | | 60 | 20 | | 20 |
| Walnut (Juglans regia L.-leafe) | Oily mass of yellowish-brownish to yellowish swamp color with a weak characteristic odor | | 20 | 20 | | 20 |
| Sage (Salvia officinalis L) - offshoots | Oily mass of greyish-brown to brownish swamp color, with wax-like inclusions, with a characteristic odor | | 20 | 20 | 30 | 10 |
| Pomegranate (Hunica granatum L.- nut) | Oily mass of greenish-yellow color | | - | 20 | 50 | 10 |
| Terpene and terpenoid content, % | | Terpene - up to 50, terpenoids - up to 10 | Terpenoids - up to 34.5 | Terpenes - up to 10.5; terpenoids - up to 27% | Terpenes up to 11.5; terpenoids - up to 21.5 | Terpenes - up to 20; terpenoi ds - up to 22.9 |

Table 4. PHP dynamics

| Group | Initial checkup | After 2 weeks | After 4 weeks | After 6 weeks | P (compared to initial) | Cleansing effect |
|---|---|---|---|---|---|---|
| #1 (Pro-Whiter Lite) | 2.04±0.19 | 1.64±0.21 | 1.53±0.19 | 1.24±0.17 | <0.05 | 39.2% |
| #2 (Pro-Whiter Classic) | 1.90±0.21 | 1.53±0.17 | 1.26±0.19 | 1.06±0.16 | <0.05 | 44.2% |
| #3 (Pro-Whiter Classic + mousse) | 1.87±0.21 | 1.45±0.16 | 1.18±0.17 | 1.01±0.17 | <0.01 | 45.9% |
| Control (placebo) | 1.96±0.22 | 1.82±0.21 | 1.79±0.20 | 1.78±0.19 | >0.5 | 9.2% |

Table 5. API dynamics

| Group | Initial checkup | After 2 weeks | After 4 weeks | After 6 weeks | P (compared to initial) | Cleansing effect |
|---|---|---|---|---|---|---|
| #1 (Pro-Whiter Lite) | 62.2±4.95 | 54.8±4.02 | 42.2±3.97 | 35.9±3.89 | <0.001 | 42.2% |
| #2 (Pro-Whiter Classic) | 49.7±4.70 | 37.8±3.86 | 29.5±3.05 | 26.1±2.70 | <0.001 | 47.4% |
| #3 (Pro-Whiter Classic+mousse ) | 64.6±6.23 | 43.2±5.06 | 38.8±5.14 | 34.5±4.98 | <0.01 | 46.6% |

(continued)

| Group | Initial checkup | After 2 weeks | After 4 weeks | After 6 weeks | P (compared to initial) | Cleansing effect |
|---|---|---|---|---|---|---|
| Control (placebo) | 54.6±5.26 | 50.7±4.32 | 49.6±3.74 | 48.8±3.26 | >0.5 | 10.6% |

Table 6. GI dynamics

| Group | Initial checkup | After 2 weeks | After 4 weeks | After 6 weeks | P (compared to initial) | Anti-inflammatory effect |
|---|---|---|---|---|---|---|
| #1 (Pro-Whiter Lite) | 0.85±0.15 | 0.64±0.13 | 0.55±0.12 | 0.48±0.12 | >0.5 | 43.5% |
| #2 (Pro-Whiter Classic) | 0.77±0.12 | 0.49±0.09 | 0.41±0.08 | 0.36±0.07 | <0.01 | 53.2% |
| #3 (Pro-Whiter Classic+mousse) | 0.68±0.12 | 0.42±0.10 | 0.34±0.08 | 0.30±0.08 | <0.01 | 55.9% |
| Control (placebo) | 0.73±0.14 | 0.70±0.12 | 0.67±0.12 | 0.67±0.10 | >0.5 | 8.2% |

Table 7. SBI dynamics

| Group | Initial checkup | After 2 weeks | After 4 weeks | After 6 weeks | P (compared to initial) | Gum bleeding reduction |
|---|---|---|---|---|---|---|
| #1 (Pro-Whiter Lite) | 29.6±6.05 | 23.2±5.96 | 20.3±5.78 | 19.4±5.91 | >0.5 | 34.4% |
| #2 (Pro-Whiter Classic) | 30.5±4.02 | 21.8±3.64 | 19.7±2.98 | 16.7±2.67 | <0.01 | 45.3% |
| #3 (Pro-Whiter Classic + mousse) | 30.2±7.03 | 18.2±5.04 | 15.6±4.78 | 13.8±4.35 | <0.05 | 54.3% |
| Control (placebo) | 27.3±5.67 | 26.8±4.74 | 26.2±4.62 | 24.8±4.42 | >0.5 | 9.1% |

**Claims**

1. Tobacco tar removal agent comprising carbon dioxide plant extracts with terpene and/or terpenoid content in the extract of min. 10 mass % obtained by supercritical extraction by natural carbon dioxide from plants including green tea, walnut, sage and fennel, and **characterized by** that it additionally comprises carbon dioxide pomegranate extract obtained by supercritical extraction by natural carbon dioxide and by that it comprises the extracts in the following proportions, mass %:

   | | |
   |---|---|
   | Pomegranate extract | 10-60 |
   | Walnut extract | 10-60 |
   | Sage extract | 10-60 |
   | Fennel extract | 10-60 |
   | Green tea extract | the rest. |

2. Composition for oral cavity hygiene **characterized by** that it comprises the tobacco tar removal agent in accordance with claim 1 in an effective quantity and an acceptable carrier.

3. Composition according to claim 2 **characterized by** that it constitutes a toothpaste.

4. Composition according to claim 2 **characterized by** that it constitutes a mouth rinse composition.

5. Composition according to claim 2 **characterized by** that it constitutes a chewing gum.

6. Toothpaste for oral cavity hygiene **characterized by** that it comprises the tobacco tar removal agent in accordance with claim 1 in an effective quantity, and an acceptable carrier comprising substances selected from the group comprising solvents, thickeners, surfactants, abrasive substances, emulgators, solubilizers, moisturizers and mixtures thereof.

7. Toothpaste according to claim 6 **characterized by** that the carrier comprises water as solvent, silicon dioxide as abrasive substance, sodium carboxy-methylcellulose as thickener, at least one moisturizer selected from the group comprising sorbitol, glycerin and polyethyleneglycol PEG-400 as moisturizer, at least one solubilizer selected from the group comprising hydrogenated castor oil and Polysorbate-20 as solubilizer, and sodium lauryl sarcosinate as surfactant, with the following proportions of components, mass %:

| | |
|---|---|
| Tobacco tar removal agent | 0.01 - 2.0 |
| Silicon dioxide | 5.0 - 60.0 |
| Sodium carboxy-methylcellulose | 0.1 - 5.0 |
| At least one moisturizer | |
| selected from the group comprising sorbitol, glycerin and polyethyleneglycol PEG-400 | 0.5 - 70 |
| At least one solubilizer selected from the group comprising hydrogenated castor oil and polysorbate-20 | 0.1-10.0 |
| Sodium lauryl sarcosinate | 0.5 - 10.0 |
| Water | the rest |

8. Toothpaste according to claim 6 **characterized by** that it additionally comprises at least one substance selected from the group comprising sweeteners, flavorants and preservation agents in a quantity not exceeding 10 mass %.

9. Toothpaste according to claim 6 **characterized by** that it additionally comprises polyvinylpyrrolidon/vinyl acetate, hydroxyapatite, menthyl lactate, isopropyl methylphenol, $CO_2$ baobab plant extract, thyme ether oil, camphor oil, lavender bioconcentrate, chlorophyll copper complex, flavorants, preservation agents, stevia extract and sucralose, with the following proportions of components, mass %:

| | |
|---|---|
| Tobacco tar removal agent | 0.01-2.0 |
| Silicon dioxide | 5.0-60.0 |
| Sodium carboxy-methylcellulose | 0.1-5.0 |
| Sorbitol | 0.5-60.0 |
| Glycerin | 0.5-60.0 |
| Polyethyleneglycol PEG-400 | 0.1-10.0 |
| Hydrogenated castor oil | 0.1-7 |
| Polysorbate-20 | 0.1-5 |
| Polyvinylpyrrolidon/vinyl acetate | max. 3 |
| Sodium lauryl sarcosinate | 0.5-10 |
| Stevia extract | max. 3 |
| Sucralose | max. 2 |
| Flavorants | max. 3 |
| Preservation agents | max. 1 |
| Hydroxyapatite | max. 20 |
| Menthyl lactate | max. 5.0 |
| Isopropyl methylphenol | max. 0.1 |
| $CO_2$ baobab plant extract | max. 3.0 |
| Thyme ether oil | max. 1.0 |
| Camphor oil | max. 1.0 |
| Lavender bioconcentrate | max. 5.0 |
| Chlorophyll copper complex | max. 5.0 |

(continued)

| Water | the rest |
| --- | --- |

10. Toothpaste according to claim 6 **characterized by** that it additionally comprises polyvinylpyrrolidon/vinyl acetate, hydroxyapatite, menthyl lactate, isopropyl methylphenol, CO2 baobab plant extract, bergamot ether oil, anise ether oil flavorants, preservation agents, colorants, stevia extract and sucralose, with the following proportions of components, mass %:

| | |
| --- | --- |
| Tobacco tar removal agent | 0.01-2 |
| Silicon dioxide | 5-60 |
| Sodium carboxy-methylcellulose | 0.1-5 |
| Sorbitol | 0.5-60 |
| Glycerin | 0.5-60 |
| Polyethyleneglycol PEG-400 | 0.1-10 |
| Hydrogenated castor oil | 0.1-7 |
| Polysorbate-20 | 0.1-5 |
| Polyvinylpyrrolidon/vinyl acetate | max. 3 |
| Sodium lauryl sarcosinate | 0.5-10 |
| Stevia extract | max. 3 |
| Sucralose | max. 2 |
| Flavorants | max. 3 38 |
| Preservation agents | max. 1 |
| Hydroxyapatite | max. 20 |
| Menthyl lactate | max. 5.0 |
| Isopropyl methylphenol | max. 0.1 |
| $CO_2$ baobab plant extract | max. 3.0 |
| Bergamot ether oil | max. 1.0 |
| Anise ether oil | max. 1.0 |
| Colorant | max. 10.0 |
| Water | the rest |

11. Foaming mouth rinse composition for oral cavity hygiene **characterized by** that it comprises the tobacco tar removal agent in accordance with claim 1 in an effective quantity, and an acceptable carrier comprising substances selected from the group comprising solvents, surfactants, foam stabilizers, emulgators, thickeners, solubilizers, moisturizers, acidity regulators, sweeteners and mixtures thereof.

12. Composition according to claim 11 **characterized by** that it comprises water as solvent, sodium lauryl sarcosinate as surfactant, carrageenan as foam stabilizer, at least one solubilizer selected from the group comprising hydrogenated castor oil and Polysorbate-20 as solubilizer, sorbitol as moisturizer, polyvinylpyrrolidon/vinyl acetate as thickener, and at least one component selected from the group comprising L-arginine and citric acid as acidity regulator, with the following proportions of components, mass %:

| | |
| --- | --- |
| Tobacco tar removal agent | 0.01-2 |
| Sodium lauryl sarcosinate | 0.1-10 |
| Carrageenan | 0.01-3 |
| Solubilizer | 0.1-7.0 |
| Sorbitol | 1.0-20.0 |
| Polyvinylpyrrolidon/vinyl acetate | 0.1-3.0 |
| At least one regulator selected from the group comprising L-arginine | 0.01-1.0 |

(continued)

| | |
|---|---|
| and citric acid | 0.01-5.0 |
| Water | the rest. |

**Patentansprüche**

1. Mittel zur Entfernung von Tabakteer, umfassend Kohlendioxid-Pflanzenextrakte mit einem Terpen- und/oder Terpenoidgehalt von mindestens 10 Masse-% in dem Extrakt, erhältlich mittels überkritische Extraktion mittels natürlichem Kohlendioxid aus Pflanzen, einschließlich grünen Tee, Walnuss, Salbei und Fenchel, und **dadurch gekennzeichnet, dass** es zusätzlich Kohlendioxid-Granatapfelextrakt umfasst, der mittels überkritischer Extraktion mittels natürlichem Kohlendioxid erhalten wurde und dass es die Extrakte in folgenden Masse-%-Anteilen umfasst:

| | |
|---|---|
| Granatapfelextrakt | 10 - 60 |
| Walnuss-Extrakt | 10 - 60 |
| Salbei-Extrakt | 10 - 60 |
| Fenchel-Extrakt | 10 - 60 |
| Extrakt von grünem Tee | der Rest. |

2. Zusammensetzung für die Mundhöhlenhygiene, **dadurch gekennzeichnet, dass** sie das Mittel zur Entfernung von Tabakteer gemäß Anspruch 1 in einer wirksamen Menge und einen akzeptablen Träger umfasst.

3. Zusammensetzung nach Anspruch 2, **dadurch gekennzeichnet, dass** sie eine Zahnpasta darstellt.

4. Zusammensetzung nach Anspruch 2, **dadurch gekennzeichnet, dass** sie eine Mundspülzusammensetzung darstellt.

5. Zusammensetzung nach Anspruch 2, **dadurch gekennzeichnet, dass** sie einen Kaugummi darstellt.

6. Zahnpasta für die Mundhöhlenhygiene, **dadurch gekennzeichnet, dass** sie das Mittel zur Entfernung von Tabakteer gemäß Anspruch 1 in einer wirksamen Menge und einen akzeptablen Träger umfasst, der Substanzen umfasst, die aus der Gruppe ausgewählt sind, umfassend Lösungsmittel, Verdickungsmittel, Tenside, abrasive Substanzen, Emulgatoren, Lösungsmittel, Feuchtigkeitsspender und Mischungen davon.

7. Zahnpasta nach Anspruch 6, **dadurch gekennzeichnet, dass** der Träger umfasst Wasser als Lösungsmittel, Siliziumdioxid als abrasive Substanz, Natriumcarboxymethylzellulose als Verdickungsmittel, mindestens einen Feuchtigkeitsspender ausgewählt aus der Gruppe umfassend Sorbit, Glycerin und Polyethylenglykol PEG-400 als Feuchtigkeitsspender, mindestens ein Lösungsmittel ausgewählt aus der Gruppe umfassend hydriertes Rizinusöl und Polysorbat-20 als Lösungsmittel und Natriumlaurylsarcosinat als Tensid mit den folgenden Masse-%-Anteilen der Komponenten:

| | |
|---|---|
| Mittel zur Entfernung von Tabakteer | 0,01 - 2,0 |
| Siliziumdioxid | 5,0 - 60,0 |
| Natriumcarboxymethylzellulose | 0,1 - 5,0 |
| mindestens ein Feuchtigkeitsspender ausgewählt aus der Gruppe umfassend Sorbit, Glycerin und Polyethylenglykol PEG-400 | 0,5 - 70 |
| mindestens ein Lösungsmittel ausgewählt aus der Gruppe umfassend hydriertes Rizinusöl und Polysorbat-20 | 0,1 - 10,0 |
| Natriumlaurylsarcosinat | 0,5 - 10,0 |
| Wasser | der Rest. |

8. Zahnpasta nach Anspruch 6, **dadurch gekennzeichnet, dass** sie zusätzlich mindestens eine Substanz umfasst ausgewählt aus der Gruppe umfassend Süßstoffe, Aromastoffe und Konservierungsmittel in einer Menge von höchstens 10 Masse-%.

9. Zahnpasta nach Anspruch 6, **dadurch gekennzeichnet, dass** sie zusätzlich umfasst Polyvinylpyrrolidon/Vinylacetat, Hydroxylapatit, Menthyllactat, Isopropylmethylphenol, $CO_2$-Baobab-Pflanzenextrakt, Thymian-Etheröl, Kampferöl, Lavendel-Biokonzentrat, Chlorophyll-Kupfer-Komplex, Aromastoffe, Konservierungsmittel, Stevia-Extrakt und Sucralose mit den folgenden Masse-%-Anteilen der Komponenten:

| | |
|---|---|
| Mittel zur Entfernung von Tabakteer | 0,01 - 2,0 |
| Siliziumdioxid | 5,0 - 60,0 |
| Natriumcarboxymethylzellulose | 0,1 - 5,0 |
| Sorbit | 0,5 - 60,0 |
| Glycerin | 0,5 - 60,0 |
| Polyethylenglykol PEG-400 | 0,1 - 10,0 |
| hydriertem Rizinusöl | 0,1 - 7 |
| Polysorbat-20 | 0,1 - 5 |
| Polyvinylpyrrolidon/Vinylacetat | max. 3 |
| Natriumlaurylsarcosinat | 0,5 - 10,0 |
| Stevia-Extrakt | max. 3 |
| Sucralose | max. 2 |
| Aromastoffe | max. 3 |
| Konservierungsmittel | max. 1 |
| Hydroxylapatit | max. 20 |
| Menthyllactat | max. 5,0 |
| Isopropylmethylphenol | max. 0,1 |
| $CO_2$-Baobab-Pflanzenextrakt | max. 3,0 |
| Thymian-Etheröl | max. 1,0 |
| Kampferöl | max. 1,0 |
| Lavendel-Biokonzentrat | max. 5,0 |
| Chlorophyll-Kupfer-Komplex | max. 5,0 |
| Wasser | der Rest. |

10. Zahnpasta nach Anspruch 6, **dadurch gekennzeichnet, dass** sie zusätzlich umfasst Polyvinylpyrrolidon/Vinylacetat, Hydroxylapatit, Menthyllactat, Isopropylmethylphenol, $CO_2$-Baobab-Pflanzenextrakt, Bergamotte-Etheröl, Anis-Etherölaromen, Konservierungsmittel, Farbstoffe, Stevia-Extrakt und Sucralose mit den folgenden Masse-%-Anteilen der Komponenten:

| | |
|---|---|
| Mittel zur Entfernung von Tabakteer | 0,01 - 2,0 |
| Siliziumdioxid | 5,0 - 60,0 |
| Natriumcarboxymethylzellulose | 0,1 - 5 |
| Sorbit | 0,5 - 60 |
| Glycerin | 0,5 - 60 |
| Polyethylenglykol PEG-400 | 0,1 - 10 |
| hydriertem Rizinusöl | 0,1 - 7 |
| Polysorbat-20 | 0,1 - 5 |
| Polyvinylpyrrolidon/Vinylacetat | max. 3 |
| Natriumlaurylsarcosinat | 0,5 - 10 |
| Stevia-Extrakt | max. 3 |
| Sucralose | max. 2 |
| Aromastoffe | max. 3 |
| Konservierungsmittel | max. 1 |
| Hydroxylapatit | max. 20 |

(fortgesetzt)

| | |
|---|---|
| Menthyllactat | max. 5,0 |
| Isopropylmethylphenol | max. 0,1 |
| $CO_2$-Baobab-Pflanzenextrakt | max. 3,0 |
| Bergamotte-Etheröl | max. 1,0 |
| Anis-Etheröl | max. 1,0 |
| Farbstoff | max. 10,0 |
| Wasser | der Rest. |

**11.** Schäumende Mundspülzusammensetzung für die Mundhöhlenhygiene, **dadurch gekennzeichnet, dass** sie das Mittel zur Entfernung von Tabakteer nach Anspruch 1 in einer wirksamen Menge und einen akzeptablen Träger umfasst, der Substanzen umfasst, die aus der Gruppe ausgewählt sind, umfassend Lösungsmittel, Tenside, Schaumstabilisatoren, Emulgatoren, Verdickungsmittel, Lösungsmittel, Feuchtigkeitsspender, Säureregulatoren, Süßungsmittel und Mischungen davon.

**12.** Zusammensetzung nach Anspruch 11, **dadurch gekennzeichnet, dass** sie umfasst Wasser als Lösungsmittel, Natriumlaurylsarcosinat als Tensid, Carrageenan als Schaumstabilisator, mindestens ein Lösungsmittel, ausgewählt aus der Gruppe umfassend hydriertes Rizinusöl und Polysorbat-20 als Lösungsmittel, Sorbit als Feuchtigkeitsspender, Polyvinylpyrrolidon/Vinylacetat als Verdickungsmittel und mindestens eine Komponente, ausgewählt aus der Gruppe umfassend L-Argininin und Zitronensäure als Säureregulator, mit den folgenden Masse-%-Anteilen der Komponenten:

| | |
|---|---|
| Mittel zur Entfernung von Tabakteer | 0,01 - 2 |
| Natriumlaurylsarcosinat | 0,1 - 10 |
| Carrageenan | 0,01 - 3 |
| Lösungsmittel | 0,1 - 7,0 |
| Sorbit | 1,0 - 20,0 |
| Polyvinylpyrrolidon/Vinylacetat mindestens ein Regulator ausgewählt aus der Gruppe umfassend | 0,1 - 3,0 |
| L-Argininin | 0,01 - 1,0 |
| und Zitronensäure | 0,01 - 5,0 |
| Wasser | der Rest. |

**Revendications**

**1.** Agent d'élimination du goudron de tabac, comprenant des extraits végétaux au dioxyde de carbone ayant une teneur en terpènes et/ou terpénoïdes dans l'extrait d'au moins 10% en masse obtenus par une extraction supercritique par du dioxyde de carbone naturel à partir de plantes comportant le thé vert, la noix, la sauge et le fenouil, et **caractérisé en ce qu'**il comprend en outre un extrait de grenade au dioxyde de carbone obtenu par une extraction supercritique par du dioxyde de carbone naturel et **en ce qu'**il comprend les extraits selon les proportions suivantes, en % en masse :

| | |
|---|---|
| Extrait de grenade | 10 - 60 |
| Extrait de noix | 10 - 60 |
| Extrait de sauge | 10 - 60 |
| Extrait de fenouil | 10 - 60 |
| Extrait de thé vert | qsp. |

**2.** Composition destinée à l'hygiène de la cavité orale, **caractérisée en ce qu'**elle comprend l'agent d'élimination du goudron de tabac selon la revendication 1 selon une quantité efficace, et un véhicule acceptable.

**3.** Composition selon la revendication 2, **caractérisée en ce qu'**elle constitue un dentifrice.

**4.** Composition selon la revendication 2, **caractérisée en ce qu'**elle constitue une composition de bain de bouche.

**5.** Composition selon la revendication 2, **caractérisée en ce qu'**elle constitue un chewing-gum.

**6.** Dentifrice destiné à l'hygiène de la cavité orale, **caractérisé en ce qu'**il comprend l'agent d'élimination du goudron de tabac selon la revendication 1 selon une quantité efficace, et un véhicule acceptable comprenant des substances choisies dans le groupe constitué par les solvants, les épaississants, les agents tensioactifs, les substances abrasives, les agents émulsifiants, les agents solubilisants, les hydratants et des mélanges de ceux-ci.

**7.** Dentifrice selon la revendication 6, **caractérisé en ce que** le véhicule comprend de l'eau comme solvant, du dioxyde de silicium comme substance abrasive, de la carboxyméthylcellulose de sodium comme épaississant, au moins un hydratant choisi dans le groupe constitué par le sorbitol, le glycérol et le polyéthylène glycol PEG-400 comme hydratant, au moins un agent solubilisant choisi dans le groupe constitué par l'huile de ricin hydrogénée et le Polysorbate-20 comme agent solubilisant, et du lauryl sarcosinate de sodium comme agent tensioactif, avec les proportions suivantes de composants, en % en masse :

| | |
|---|---|
| Agent d'élimination du goudron de tabac | 0,01-2,0 |
| Dioxyde de silicium | 5,0-60,0 |
| Carboxyméthylcellulose de sodium | 0,1-5,0 |
| Au moins un agent hydratant choisi dans le groupe constitué par le sorbitol, le glycérol et le polyéthylène glycol PEG-400 | 0,5-70 |
| Au moins un agent solubilisant choisi dans le groupe constitué par l'huile de ricin hydrogénée et le Polysorbate-20 | 0,1-10,0 |
| Lauryl sarcosinate de sodium | 0,5-10,0 |
| Eau | qsp |

**8.** Dentifrice selon la revendication 6, **caractérisé en ce qu'**il contient en outre au moins une substance choisie dans le groupe constitué par les édulcorants, les aromatisants et les agents conservateurs, selon une quantité non supérieure à 10% en masse.

**9.** Dentifrice selon la revendication 6, **caractérisé en ce qu'**il contient en outre de la polyvinylpyrrolidone/ acétate de vinyle, de l'hydroxyapatite, du lactate de menthyle, de l'isopropyl méthylphénol, un extrait de plante de baobab au $CO_2$, une huile d'éther de thym, de l'huile de camphre, un bioconcentré de lavande, un complexe de cuivre-chlorophylles, des aromatisants, des agents conservateurs, un extrait de stévia et du sucralose, avec les proportions suivantes de composants, en % en masse :

| | |
|---|---|
| Agent d'élimination du goudron de tabac | 0,01-2,0 |
| Dioxyde de silicium | 5,0-60,0 |
| Carboxyméthylcellulose de sodium | 0,1-5,0 |
| Sorbitol | 0,5-60,0 |
| Glycérol | 0,5-60,0 |
| Polyéthylène glycol PEG-400 | 0,1-10,0 |
| Huile de ricin hydrogénée | 0,1-7 |
| Polysorbate-20 | 0,1-5 |
| Polyvinylpyrrolidone/acétate de vinyle | max. 3 |
| Lauryl sarcosinate de sodium | 0,5-10,0 |
| Extrait de stévia | max. 3 |
| Sucralose | max. 2 |
| Aromatisants | max. 3 |
| Agents conservateurs | max. 1 |
| Hydroxyapatite | max. 20 |
| Lactate de menthyle | max. 5,0 |
| Isopropyl méthylphénol | max. 0,1 |

(suite)

| | |
|---|---|
| Extrait de plante de baobab au $CO_2$ | max. 3,0 |
| Huile d'éther de thym | max. 1,0 |
| Huile de camphre | max. 1,0 |
| Bioconcentré de lavande | max. 5,0 |
| Complexe de cuivre-chlorophylles | max. 5,0 |
| Eau | qsp |

10. Dentifrice selon la revendication 6, **caractérisé en ce qu'**il contient en outre de la polyvinylpyrrolidone/ acétate de vinyle, de l'hydroxyapatite, du lactate de menthyle, de l'isopropyl méthylphénol, un extrait de plante de baobab au $CO_2$, une huile d'éther de bergamote, une huile d'éther d'anis, des aromatisants, des agents conservateurs, des colorants, un extrait de stévia et du sucralose, avec les proportions suivantes de composants, en % en masse :

| | |
|---|---|
| Agent d'élimination du goudron de tabac | 0,01-2 |
| Dioxyde de silicium | 5-60 |
| Carboxyméthylcellulose de sodium | 0,1-5 |
| Sorbitol | 0,5-60 |
| Glycérol | 0,5-60 |
| Polyéthylène glycol PEG-400 | 0,1-10 |
| Huile de ricin hydrogénée | 0,1-7 |
| Polysorbate-20 | 0,1-5 |
| Polyvinylpyrrolidone/acétate de vinyle | max. 3 |
| Lauryl sarcosinate de sodium | 0,5-10 |
| Extrait de stévia | max. 3 |
| Sucralose | max. 2 |
| Aromatisants | max. 3 38 |
| Agents conservateurs | max. 1 |
| Hydroxyapatite | max. 20 |
| Lactate de menthyle | max. 5,0 |
| Isopropyl méthylphénol | max. 0,1 |
| Extrait de plante de baobab au $CO_2$ | max. 3,0 |
| Huile d'éther de bergamote | max. 1,0 |
| Huile d'éther d'anis | max. 1,0 |
| Colorant | max. 10,0 |
| Eau | qsp |

11. Composition de bain de bouche moussant destinée à l'hygiène de la cavité orale, **caractérisée en ce qu'**elle comprend l'agent d'élimination du goudron de tabac selon la revendication 1 selon une quantité efficace, et un véhicule acceptable comprenant des substances choisies dans le groupe constitué par les solvants, les agents tensioactifs, les stabilisateurs de mousse, les agents émulsifiants, les épaississants, les agents solubilisants, les hydratants, les régulateurs d'acidité, les édulcorants, et des mélanges de ceux-ci.

12. Composition selon la revendication 11, **caractérisée en ce qu'**elle comprend de l'eau comme solvant, du lauryl sarcosinate de sodium comme agent tensioactif, un carraghénane comme stabilisateur de mousse, au moins un agent solubilisant choisi dans le groupe constitué par l'huile de ricin hydrogénée et le Polysorbate-20 comme agent solubilisant, du sorbitol comme hydratant, de la polyvinylpyrrolidone/acétate de vinyle comme épaississant, et au moins un composant choisi dans le groupe constitué par la L-arginine et l'acide citrique comme régulateur d'acidité, avec les proportions suivantes de composants, en % en masse :

| | |
|---|---|
| Agent d'élimination du goudron de tabac | 0,01-2 |
| Lauryl sarcosinate de sodium | 0,1-10 |

(suite)

| | |
|---|---|
| Carraghénane | 0,01-3 |
| Agent solubilisant | 0,1-7,0 |
| Sorbitol | 1,0-20,0 |
| Polyvinylpyrrolidone/acétate de vinyle | 0,1-3,0 |
| Au moins un régulateur choisi dans le groupe constitué par la L-arginine | 0,01-1,0 |
| et l'acide citrique | 0,01-5,0 |
| Eau | qsp. |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003335646 B
- JP 2002047157 B
- JP 2001199855 B
- JP 2006104101 B
- US 5662888 A
- CN 1864661 A
- RU 2310436

- RU 2306922
- CN 1172647 A
- WO 2007109802 A2
- WO 2004004650 A2
- JP 62181212 A
- JP 2005194526 A
- RU 2203032 C1

**Non-patent literature cited in the description**

- Separation of Catechins from Green Tea Using Carbon Dioxide Extraction. **CHANG CHIEHMING et al.** FOOD CHEMISTRY. ELSEVIER LTD, vol. 68

- Database. 1107762